# EUROPEAN PATENT SPECIFICATION

(11) **EP 4 103 769 B1**
(45) Date of publication and mention of the grant of the patent: **22.07.2026**
(21) Application number: 21709099.2
(22) Date of filing: 12.02.2021
(51) Int. Cl.: D01D 5/00, A61K 8/02, A61K 8/73, A61Q 19/00, B32B 27/12, D01F 1/10, A61L 27/54, D04H 1/728, A61L 15/22, A61L 15/42, A61L 15/44

(54) **NATURAL COMPOSITION BASED ON POLYMERS TO BE ELECTROSPUN, AND METHOD TO PREPARE THE SAME**
NATÜRLICHE ZUSAMMENSETZUNG AUF DER GRUNDLAGE VON ELEKTROSPUN-POLYMEREN UND VERFAHREN ZUR VORBEREITUNG DER GLEICHEN
COMPOSITION NATURELLE BASÉE SUR DES POLYMÈRES ÉLECTROFILABLES ET PROCÉDÉ DE PRÉPARATION DES MÊMES

(30) Priority: 13.02.2020 IT 202000002827; 05.01.2021 IT 202100000089
(43) Date of publication of application: 21.12.2022
(73) Proprietor: Bakel S.P.A., 33100 Udine (IT)
(72) Inventor: GREGORIS, Raffaella, 33100 Udine (IT); MANFREDINI, Stefano, 44049 Vigarano Mainarda (IT); VERTUANI, Silvia, 44123 Ferrara (IT)
(74) Representative: Petraz, Gilberto Luigi
(86) International application number: PCT/IB2021/051190
(87) International publication number: WO 2021/161250

(56) References cited:
- CN-A- 109 589 318
- GB-A- 2 484 319
- US-A1- 2006 264 130
- US-A1- 2017 251 789
- ZE-YU QIN ET AL.: "Fast dissolving oral films for drug delivery prepared from chitosan/ pullulan electrospinning nanofibers", INTERNATIONAL JOURNAL OF BIOLOGICAL MACROMOLECULES, vol. 137, 15 September 2019 (2019-09-15), pages 224 - 231, XP085790804, ISSN: 0141-8130, Retrieved from the Internet <URL:https://www.sciencedirect.com/science/article/pii/S0141813019339170> [retrieved on 20250521], DOI: 10.1016/j.ijbiomac.2019.06.224
- SHIH-CHUAN LIU ET AL.: "Electrospun Food-Grade Ultrafine Fibers from Pectin and Pullulan Blends", FOOD AND NUTRITION SCIENCES, vol. 7, no. 7, 29 June 2016 (2016-06-29), pages 636 - 646, XP055706253, ISSN: 2157-944X, Retrieved from the Internet <URL:https://www.scirp.org/journal/paperinformation?paperid=67821> [retrieved on 20250521], DOI: 10.4236/fns.2016.77065
- PING SHAO ET AL.: "Fabrication and characterization of tea polyphenols loaded pullulan-CMC electrospun nanofiber for fruit preservation", INTERNATIONAL JOURNAL OF BIOLOGICAL MACROMOLECULES, vol. 107, 15 December 2017 (2017-12-15), pages 1908 - 1914, XP093106624, Retrieved from the Internet <URL:https://www.sciencedirect.com/science/article/pii/S0141813017330726> [retrieved on 20250521], DOI: 10.1016/j.ijbiomac.2017.10.054

## Description

### FIELD OF THE INVENTION

The present invention concerns a composition to be electrospun, that is, which allows to produce nanofibers by electrospinning. More particularly, the composition is the type based on a polymer, preferably a biocompatible polymer, to be electrospun.

### BACKGROUND OF THE INVENTION

The electrospinning process is known, which allows to obtain nanofibers, that is, continuous fibers with a diameter in the order of a nanometer, starting from a composition based on a polymer compound to be electrospun that is subjected to an electric field. Depending on the type of compound that is electrospun, the nanofibers obtained can then have applications in any field whatsoever, for example in medicine, military defense, environment, biotechnology, energy or in the cosmetic field.

For environmental reasons, electrospinning tends to be performed using ecological solvents, in particular in water. However, electrospinning polymers in pure water is not easy due to its surface tension and the viscosity of the compound that is obtained.

To overcome this problem, attempts have been made to lower the surface tension and viscosity of the water, for example by electrospinning in aqueous solutions of ammonium, therefore in solutions with a high pH, or also in water in the presence of dimethylformamide DMF at 40°C. Experiments have also been performed with hexafluoroisopropanol HFIP or ethanol. By proceeding in this way, however, the ecological aspect is compromised.

Some alternative solutions have been proposed. For example, CN 109589318 discloses a composite film made of fucus polysaccharide, sodium alginate and phyla nodiflora nano cellulose as raw materials, to be used for accelerating wound healing. Such document does not teach to produce electrospun fibers.

US 2006/264130 describes a method to produce nanometric fibers by electrospinning a composition comprising a polysaccharide and an active ingredient. The composition can comprise any polysaccharide or mixture of polysaccharides provided that they are soluble or dispersible in water. This document also describes the addition of ethanol to help the electrospinning of a polysaccharide.

GB 2484319 discloses an electrospun fiber composed of a polymer selected from alginate, chitosan, pectin, polyethylene oxide (PEO), poly-lactic-acid (PLA, PLLA), polyethylene terephthalate (PET) or polypropylene (PP) and honey as an active ingredient. The fibers are used in wound dressings, or as tissue or scaffold on which living cells could be grown in vitro or in vivo.

The article "Fast dissolving oral films for drug delivery prepared from chitosan/pullulan electrospinning nanofibers" by Qin et al, Science Direct, vol. 137, p. 224-231 discusses the mixing of pullulan with chitosan for the production of nanofibers, and verifies the influence of chitosan addition on electrospun pullulan fibers.

The article "Electrospun Food-Grade Ultrafine Fibers from Pectin and Pullulan Blends" by Liu et al, Food and Nutrition Sciences, vol. 7, n. 7, concerns the formation of food-grade pectin and pullulan nanofibers for future food applications.

The article "Fabrication and characterization of tea polyphenols loaded pullulan-CMC electrospun nanofiber for fruit preservation" by Shao et al, International Journal of Biological Macromolecules, vol. 107, p. 1908-1914, describes a pullulan:CMC 90:10 composition for electrospinning, with polyphenols added at different percentages. Some conditions of the electrospinning phase are varied (voltage and feed rate of the composition to the needle) to verify their influence on the morphology of the fibers obtained, in particular on the diameter of the fibers and the probability of accumulation formation. It is also indicated that the concentration of polyphenols has an influence on the diameter of the fibers.

However, even these solutions do not fully satisfy the needs of the sector with regard to the surface tension of water as a solvent, and the viscosity of the solution obtained.

There is therefore a need to perfect a composition to be electrospun which can overcome at least one of the disadvantages of the state of the art.

In particular, one purpose of the present invention is to provide a composition to be electrospun that can be electrospun in pure water or aqueous solutions that have no environmental impact.

Another purpose of the present invention is to provide a composition to be electrospun which allows to produce continuous nanofibers, or in any case fibers, with an almost constant diameter and free from defects.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, this application describes a composition to be electrospun that overcomes the limits of the state of the art and eliminates the defects present therein.

In accordance with some embodiments, the composition comprises hyaluronic acid as a first compound to be electrospun and a spinning promoter. The spinning promoter has the function of facilitating the spinning of the first compound, in particular of establishing the electrospinning method so as to obtain regular fibers.

The spinning promoter is selected from pullulan and alginate. The promoter can also comprise a mixture of pullulan and alginate.

According to some embodiments, the composition also comprises an active ingredient. This active ingredient can, for example, be selected from cosmetic active ingredients, pharmaceutical active ingredients and nutritional active ingredients.

According to some embodiments, the composition also comprises a stabilizer, suitable to improve the stability of the fibers obtained following the electrospinning. Preferably, the stabilizer is a cross-linkable polymer.

One advantage of the composition as above lies in the possibility of making a cosmetic, pharmaceutical or nutritional product based on natural polymers, with topical concentrations of compound of interest much higher than those obtainable with traditional formulations. This is due to the fact that the compound of interest is precisely integrated into the very structure of the final product. With known compositions, it is practically impossible to reach high concentrations, even with polysaccharides, collagen, gelatin, albumin, elastin and their low molecular weight derivatives, since the viscosity of the product increases too much and it is not possible to exceed 5-10% by weight. With the present composition, it is possible to obtain products that allow to apply concentrations of up to 50% by weight of the compound of interest.

According to one aspect, a method to prepare a composition to be electrospun is also provided, in which a first compound to be electrospun and a spinning promoter are mixed.

The first compound to be electrospun is hyaluronic acid HA.

The spinning promoter is selected from pullulan and alginate. The promoter can also comprise a mixture of pullulan and alginate.

### DETAILED DESCRIPTION OF SOME EMBODIMENTS

We will now refer in detail to the possible embodiments of the invention, of which one or more examples are shown in the attached drawings, as a non-limiting example. The phraseology and terminology used here is also for the purposes of providing non-limiting examples.

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

All measurements are carried out, unless otherwise indicated, at 25°C (room temperature) and at atmospheric pressure. All temperatures, unless otherwise indicated, are expressed in degrees Celsius.

All percentages and ratios indicated here are understood to refer to the weight of the total composition (w/w), unless otherwise indicated.

All percentage intervals reported here are supplied with the provision that the sum with respect to the overall composition is 100%, unless otherwise indicated.

All the intervals reported here shall be understood to include the extremes, including those that report an interval "between" two values, unless otherwise indicated.

The present description also includes the intervals that derive from overlapping or uniting two or more intervals described, unless otherwise indicated.

The present description also includes the intervals that can derive from the combination of two or more values taken at different points, unless otherwise indicated.

Where water is mentioned, we mean distilled water, unless otherwise specified.

The composition comprises a first compound to be electrospun.

In the present invention, the first compound to be electrospun is hyaluronic acid.

Referenced here as comparative examples of known state of the art, a first compound to be electrospun has conventionally been a biocompatible polymer suitable to be electrospun selected from xanthan gum, pectin, chitin, chitosan, dextran, carrageenan, guar gum, agar, hydroxypropyl methylcellulose HPMC, hydroxypropyl cellulose HPC, hydroxyethyl cellulose HEC, sodium carboxymethyl cellulose Na-CMC, albumin, starch, gelatin, collagen, elastin, β-glucans, chondroitin sulfate, dermatan sulfate, heparin, heparan sulfate, galactomannans, xylans, gum arabic, gum ghatti, glucomannan, acemannan, soluble dietary fibers, glycogen, amylose and polysaccharides derived from plants, bacteria and fungi and their derivatives.

These compounds or classes of compounds have, as properties, the possibility of modifying the viscosity of liquids, which makes them suitable to form regular electrospun fibers with good mechanical and absorption properties. They are also all biocompatible, of natural origin, and can be used in the food, pharmaceutical and/or cosmetic fields.

In particular, xanthan gum, dextran, carrageenan, Na-CMC, starch, gelatin and gum ghatti are used as a thickener, stabilizer and possibly also as a gelling agent in the food sector. In addition, xanthan gum is used as a stabilizer for suspensions and emulsions in the pharmaceutical and cosmetic fields, guar gum is used as a thickener and gelling agent also in the pharmaceutical and cosmetic fields, carrageenan is used as an inactive excipient in the pharmaceutical field. Dextran is also used as a thickener in the pharmaceutical field.

Chitosan is used in the food sector, in low-calorie diets, and in the pharmaceutical field as an excipient, in particular for products to be inhaled. On the other hand, pectin is used as a gelling agent in the food sector and as a dietary and probiotic agent in the pharmaceutical field.

Agar, galactomannans and glucomannan are used as a gelling agent in the nutritional field.

Among the cellulose derivatives, HPMC is used as a stabilizer and viscosity regulator in the food sector, and as collyrium or excipient for oral medicines in the pharmaceutical field. HPC is used as a food additive, and as collyrium or binder for tablets in the pharmaceutical field. HEC is used as a thickener and gelling agent in the pharmaceutical and cosmetic fields.

β-glucans are used as dietary fibers, as are xylans. Chondroitin sulfate is used as a food supplement, and also in the treatment of osteoarthritis symptoms. Dermatan sulfate, heparin and heparan sulfate are known as anticoagulants in the pharmaceutical field.

Gum arabic is used in the food industry as a stabilizing excipient and viscosity modifier. Acemannan, on the other hand, is known in the pharmaceutical field for its immunostimulant properties.

It should be noted that some of these comparative compounds have their own functions in the cosmetic, pharmaceutical or food sectors, such as for example starch, elastin, hyaluronic acid, heparin, collagen, pectin, β-glucans, chondroitin sulfate, dermatan sulfate, heparan sulfate and their derivatives, among others.

In the present invention, however, the first compound to be electrospun is hyaluronic acid, and it can be of the linear or cross-linked type, and can have a high mass, for example in the order of a million Dalton or even more, or alternatively have a low mass, typically in the order of 10,000 Dalton or less. It is also possible to provide a mixture of linear hyaluronic acid with cross-linked hyaluronic acid, so as to modulate the rigidity of the yarn that will be obtained, as well as the three-dimensional structure of a film that can be obtained by depositing the yarn on a support.

The hyaluronic acid first compound is advantageously diluted in an aqueous or aqueous-based solution, at low concentrations, for example between 0.1% and 10% by weight, preferably 0.5% and 5% by weight, more preferably between 0.6% and 2.5% by weight.

The composition to be electrospun also comprises a spinning promoter which is a carrier polymer without filler, favorably biocompatible. It is selected from alginate and pullulan. The spinning promoter is preferably pullulan, since it allows to obtain the best results.

Pullulan can be diluted in an aqueous or aqueous-based solution preferably at a concentration comprised between 1% and 40%, preferably between 3% and 30%, more preferably between 10% and 20% by weight.

The first compound to be electrospun and the promoter are mixed in proportions (first compound):promoter preferably comprised between 10:1 and 1:10, more preferably between 4:1 and 1:7, even more preferably between 3:1 and 1:6 by weight.

The composition also comprises at least one active ingredient, of the pharmaceutical, nutritional and/or cosmetic type.

It should be noted that the active ingredients can have various types of function, regardless of their field of action.

The cosmetic active ingredient can be of the following types: anti-seborrheic (e.g. sebacic acid, azelaic acid), anti-sebum (e.g. coal powder), antimicrobial (e.g. climbazole, piroctone olamine), antioxidant (e.g. ascorbic acid, tocopherol, coenzyme Q10, resveratrol, glutathione), antiperspirant (e.g. aluminum chlorohydrate, aluminum sesquichlorohydrate), astringent (e.g. Citrus aurantifolia flower extract, calcium lactate), whitener (e.g. glabridin, ammonium persulfate) , make-up remover (e.g. sodium cocoyl glutamate), deodorant (e.g. triethyl citrate, zinc ricinoleate), exfoliant (e.g. glycolic acid, malic acid, mandelic acid), flavorings (e.g. citral, honey), fragrance (e.g. d, 1-limonene, coumarin), humectant (e.g. glycerin, propanediol), keratolytic (e.g. chloroacetic acid, salicylic acid), moisturizing (e.g. Aloe arborescens leaf extract), perfuming (e.g. geraniol, linalool), emollient (e.g. triolein, squalene), refreshing (e.g. menthol, menthyl lactate), skin moisturizer (e.g. panthenol, allantoin), skin protection (e.g. sphingolipids, zinc oxide), smoothing (e.g. Ricinus communis seed oil), soothing (e.g. Hamamelis virginiana extracts, Chamomile recutita extracts, bisabolol) or tonic (e.g. arnica montana, Capsicum frutescens extract), UV filter (e.g. methylene bis-benzotriazolyl tetramethylbutylphenol, ethylhexyl methoxycinnamate, caffeine, theine, theobromine, theophylline).

The active pharmaceutical ingredient can be of the following types: 5-alpha-reductase inhibitor (e.g. finasteride), 5-aminosalicylates (e.g. mesalamine), 5HT3 receptor antagonist (e.g. ondansetron), ACE inhibitor with calcium channel blocker (e.g. amlodipine/benazepril), ACE inhibitor with thiazides (e.g. hydrochlorothiazide), adamantane antivirals (e.g. amantadine), adrenal corticosteroid inhibitor (e.g. aminoglutethimide), adrenergic bronchodilators (e.g. albuterol), hypertensive emergencies agent (e.g. diazoxide) pulmonary hypertension agent (e.g. treprostinil), aldosterone receptor antagonist (e.g. spironolactone), alkylating agent (e.g. cyclophosphamide), allergenic (e.g. house dust mite allergen extracts), alpha-glucosidase inhibitor (e.g. miglitol), amebicides (e.g. metronidazole), aminoglycosides (e.g. tobramiycin), aminopenicillins (e.g. amoxicillin), aminosalicylates (e.g. aminosalicylic acid), AMPA receptor antagonist (e.g. perampanel), amylin analogues (e.g. pramlintide), analgesics (e.g. acetaminophen), androgenic and anabolic steroids (e.g. testosterone), enzyme inhibitor converting angiotensin (e.g. ramipril), angiotensin II inhibitor with calcium channel blocker (e.g. amlodipine/olmesartan), angiotensin II inhibitor with thiazides (e.g. hydrochlorothiazide/olmesartan), angiotensin receptor blockers (e.g. valsartan), inhibitor of angiotensin and neprilysin receptor blockers (e.g. sacubitril/valsartan), anorectal preparations (e.g. hydrocortisone/pramoxin), anorexiants (e.g. phentermine), antacids (e.g. magnesium hydroxide), anthelmintics (e.g. pyrantel), anti-angiogenic ophthalmic agent (e.g. aflibercept), anti-CTLA-4 monoclonal antibody (e.g. ipilimumab), anti-PD-1 monoclonal antibody (e.g. nivolumab), antiadrenergic agent (central) with thiazides (e.g. hydrochlorothiazide/methyldopa), antiadrenergic agent (peripheral) with thiazides (e.g. polythiazide/prazosin), centrally acting antiadrenergic agent (e.g. guanfacine), peripherally acting antiadrenergic agent (e.g. tamsulosin), antiandrogens (e.g. enzalutamide), antianginal agent (e.g. nitroglycerin, for example diphylline/guaifenesin), antibiotics (e.g. metronidazole), antibiotics/antineoplastics (e.g. doxorubicin), anticholinergic antiemetics (e.g. diphenhydramine), anticholinergic antiparkinsonian agent (e.g. procyclidine), anticholinergic bronchodilators (e.g. tiotropium), for example anticholinergics/antispasmodics (e.g. hyoscyamine), anticoagulant agent (e.g. phytonadione), anticonvulsants (e.g. lacosamide), antidepressant (e.g. bupropion), antidiarrheal (e.g. loperamide), antidiuretic hormone (e.g. desmopressin), antidote (e.g. naltrexone dronabinol), antifungal (e.g. griseofulvin), antigonadotropic agent (e.g. g. danazol), antigout agent (e.g. colchicine), antihistamine (e.g. cetirizine), anti-hyperlipidemic agent and combinations (e.g. ezetimibe/simvastatin), antihyperuricemic agent (e.g. febuxostat), antimalarial (e.g. doxycycline), antimalarial, antimalarial quinoline combination (e.g. hydroxychloroquine), antimanic agent (e.g. lithium), antimetabolite (e.g. capecitabine), anti-migraine agent (e.g. rizatriptan), antineoplastic (e.g. isotretinoin), antineoplastic combination (e.g. letrozole/ribociclib), antineoplastic detoxifying agent (e.g. amifostine), antineoplastic interferon (e.g. interferon alfa-2b), antipseudomonal penicillin (e.g. carbenicillin), antipsoriatic (e.g. acitretin), antipsychotic agent (e.g. haloperidol), antirheumatic (e.g. adalimumab), antiseptic and germicidal agent (e.g. potassium iodide), antitoxin and antiviral (e.g. antivenin (crotalidae) polyvalent), antitussive (e.g. dextromethorphan), antiviral booster (e.g. ritonavir), antiviral interferon (e.g. peginterferon alfa-2a), aromatase inhibitor (e.g. anastrozole), atypical antipsychotic (e.g. aripiprazole), azole antifungal (e.g. fluconazole), bacterial vaccine (e.g. 13-valent pneumococcal vaccine), barbiturate anticonvulsant (e.g. primidone), barbiturate (e.g. phenobarbital), BCR-ABL tyrosine kinase (e.g. imatin), anticonvulsant benzodiazepine (e.g. diazepam), benzodiazepine (e.g. clonazepam), beta blocker with thiazides (e.g. bisoprolol/hydrochlorothiazide), beta-lactamase inhibitor (e.g. clavulanic acid), bile acid sequestering agent (e.g. colesevelam), bisphosphonate (e.g. zoledronic acid), BTK inhibitor (e.g. ibrutinib), calcimimetic (e.g. cinacalcet), calcineurin inhibitor (e.g. tacrolimus), calcitonin, calcium channel blocker agent (e.g. verapamil), anticonvulsant carbamate (e.g. felbamate), carbapenem (e.g. doripenem), carbapenem/beta-lactamase inhibitor (e.g. meropenem/vaborbactam), anticonvulsant carbonic anhydrase inhibitor (e.g. topiramate), carbonic anhydrase inhibitor (e.g. acetazolamide), cardiac stressing agents (e.g. regadenoson), cardio-selective beta-blockers (e.g. nebivolol), catecholamines (e.g. epinephrine), CD20 monoclonal antibody (e.g. ocrelizumab), CD30 monoclonal antibody (e.g. brentuximab), CD33 monoclonal antibody (e.g. gemtuzumab), CD38 monoclonal antibody (e.g. CD52 monoclonal), (e.g. alemtuzumab), CDK 4/6 inhibitor (e.g. palbociclib), cephalosporins/beta-lactamase inhibitor (e.g. avibactam/ceftazidime), cerumenolytics (e.g. carbamide peroxide), combination of CFTR (e.g. ivacaftor/lumacaftor), CFTR enhancer (e.g. ivacaftor), CGRP inhibitor (e.g. erenumab), chelating agent (e.g. deferasirox), chemokine receptor antagonist (e.g. maraviroc), chloride channel activator (e.g. lubiprostone), cholesterol absorption inhibitor (e.g. ezetimibe), cholinergic agonist (e.g. cevimeline), cholinergic muscle stimulants (e.g. pyridostigmine), cholinesterase inhibitor (e.g. donepezil), central nervous system stimulant (e.g. Phentermine), colony stimulating factor (e.g. Filgrastim), contraceptive (e.g. Levonorgestrel), corticotropin, coumarins and indandione (e.g. Warfarin), cox-2 inhibitor (e.g. Celecoxib), decongestant (e.g. Pseudoephedrine), diarylquinoline, dibenzazepine anticonvulsant (e.g. carbamazepine), digestive enzyme (e.g. lactase), dipeptidyl peptidase 4 inhibitor (e.g. sitagliptin), dopaminergic antiparkinsonian agent (e.g. ropinirole), drug used in alcohol dependence (e.g. acamprosate), echinocandin (e.g. caspofungin) inhibitor (e.g. erlotinib), estrogen receptor antagonist (e.g. fulvestrant), estrogen (e.g. estradiol), expectorant (e.g. guaifenesin), factor Xa inhibitor (e.g. rivaroxaban), fatty acid derivative anticonvulsant (e.g. divalproex sodium), fibric acid derivative (e.g. fenofibrate), first generation cephalosporins (e.g. cephalexin), fourth generation cephalosporins (e.g. cefepime), gallstone solubilizing agent (e.g. ursodiol), gamma-aminobutyric acid analogue (e.g. gabapentin), gamma-aminobutyric acid re-uptake inhibitor (e.g. tiagabine), general anesthetic (e.g. propofol), GI stimulant (e.g. metoclopramide), glucocorticoids (e.g. budesonide), glucose elevating agent (e.g. glucagon), glycopeptide antibiotic (e.g. vancomycin), glycoprotein platelet inhibitor (e.g. tirofiban), glycylcycline (e.g. tigecycline), gonadotropin-releasing hormone (e.g. leuprolide), gonadotropin-releasing hormone antagonist (e.g. elagolix), gonadotropin (e.g. chorionic gonadotropin) group I antiarrhythmic (e.g. phenytoin), group II antiarrhythmic (e.g. propranolol), group III antiarrhythmic (e.g. dronedarone), group IV antiarrhythmic (e.g. verapamil), group V antiarrhythmic (e.g. digoxin), growth hormone receptor blocker (e.g. pegvisomant), growth hormone (e.g. somatropin), guanylate cyclase-C agonist (e.g. linaclotide), H. pylori eradication agent (e.g. bismuth subcitrate potassium/metronidazole/tetracyclines), H2 antagonist (e.g. ranitidine), hedgehog pathway inhibitor (e.g. vismodegib), heparin antagonist (e.g. protamine), HER2 inhibitor (e.g. neratinib), herbal-based product (e.g. 5-hydroxytryptophan, aloe vera), histone deacetylase inhibitor (e.g. romidepsin), hormone/antineoplastic (e.g. medroxyprogesterone), hydantoin anticonvulsant (e.g. phenytoin), hydrazide derivative (e.g. isoniazid), immunoglobulin, impotence agent (e.g. sildenafil), incretin mimetic (e.g. liraglutide), inotropic agent (e.g. digoxin), insulin and derivatives (e.g. insulin glargine), insulin-like growth factor (e.g. mecasermin), interferon (e.g. interferon beta-1a), interleukin inhibitor (e.g. dupilumab), interleukin (e.g. aldesleukin), iron product (e.g. ferrous sulfate), ketolide (e.g. telithromycin), laxative (e.g. bisacodyl), leprostatic (e.g. clofazimine), leukotriene modifier (e.g. montelukast), lincomycin derivative (e.g. clindamycin), loop diuretic (e.g. furosemide), enzyme lysosomal (e.g. imiglucerase), macrolide (e.g. azithromycin), mast cell stabilizer (e.g. cromolyn), meglitinide (e.g. repaglinide), melanocortin receptor agonist (e.g. bremelanotide), methylxanthine (e.g. theocorticus) mineral corticoid (e.g. fludrocortisone), mineral and electrolyte (e.g. citric acid/potassium citrate), various antivirals (e.g. baloxavir marboxil), various anxiolytics, sedatives and hypnotics (e.g. zolpidem), various bone resorption inhibitors (e.g. denosumab), various cardiovascular agents (e.g. midodrine), various central nervous system agents (e.g. dalfampridine), various coagulation modifiers (e.g. tranexamic acid), various diuretics (e.g. pamabrom), various agents of the genitourinary tract (e.g. phenazopyridine), various gastrointestinal agents (e.g. misoprostol), various metabolic agents (e.g. burosumab), various respiratory agents (e.g. alpha 1-proteinase inhibitor), various topical agents (e.g. sodium hyaluronate), various vaginal agents (e.g. estradiol), mitotic inhibitor (e.g. vincristine), monoamine oxidase inhibitor (e.g. phenelzine), mouth and throat product (e.g. fluoride), mTOR inhibitor (e.g. everolimus), mucolytic (e.g. for example acetylcysteine), multikinase inhibitor (e.g. sorafenib), combination of narcotic analgesics (e.g. buprenorphine/naloxone), narcotic analgesic (e.g. fentanyl), natural penicillin (e.g. penicillin v potassium), neuraminidase inhibitor (e.g. oseltamivir), neuronal potassium channel openers (e.g. ezogabine), new generation cephalosporins (e.g. ceftaroline), NHE3 inhibitor (e.g. ceftaroline), nicotinic acid derivative (e.g. ethionamide), NK1 receptor antagonist (e.g. aprepitant), NNRTI (e.g. efavirenz), non-cardioselective beta blocker (e.g. carvedilol), not sulfonylureas (e.g. metformin), non-steroidal anti-inflammatory drug (e.g. diclofenac), NS5A inhibitor (e.g. daclatasvir), nucleoside reverse transcriptase inhibitor (NRTI) (e.g. tenofovir), nutraceutical product (e.g. omega-3 polyunsaturated fatty acids), oral food supplement (e.g. arginine), other immunostimulants (e.g. glatiramer), other immunosuppressants (e.g. omalizumab), oxazolidinedione anticonvulsant (e.g. trimethadione), oxazolidinedione antibiotic (e.g. linezolid), parathormone and analogues (e.g. for example teriparatide), PARP inhibitor (e.g. niraparib), PCSK9 inhibitor (e.g. evolocumab), penicillin resistant penicillinase (e.g. oxacillin), peripheral opioid receptor antagonist (e.g. naloxegol), mixed peripheral opioid receptor agonists (egonist/eluxadoline antagonist), peripheral vasodilator (e.g. isoxsuprine), peripherally acting antiobesity agent (e.g. orlistat), phenothiazine antiemetic (e.g. promethazine), phenothiazine antipsychotic (e.g. prochothiazine), phenylpiperazine antidepressant (e.g. trazodone), potassium phosphate inhibitor (e.g. trazodone) (e.g. idelalisib), platelet aggregation inhibitor (e.g. aspirin), platelet stimulating agent (e.g. eltrombopag), polyene (e.g. nystatin), potassium-sparing diuretic (e.g. spironolactone), probiotic (e.g. lactobacillus acidophilus), progesterone receptor modulator (e.g. ulipristal), progestin levonorgestrel), prolactin inhibitor (e.g. cabergoline), protease inhibitor (e.g. telaprevir), protease-activated receptor-1 antagonist (e.g. vorapaxar), proteasome inhibitor (e.g. bortezomib), proton pump inhibitor (e.g. omeprazole), psoralen (e.g. methoxsalen), purine nucleoside (e.g. valaciclovir), pyrrolidine anticonvulsant (e.g. levetiracetam), quinolones (e.g. ciprofloxacin), recombinant human erythropoietin (e.g. epoetin alfa) renin inhibitor (e.g. aliskiren), rifamycin derivative (e.g. rifampicin), salicylate (e.g. aspirin), second generation cephalosporin (e.g. selective cefuroxime receptor), modulator (e.g. ospemifene), selective immunosuppressant (e.g. natalizumab), phosphodiesterase-4 selective inhibitor (e.g. roflumilast), selective serotonin reuptake inhibitor (e.g. escitalopram), serotonin-norepinephrine reuptake inhibitor (e.g. duloxetine), serotoninergic neuroenteric modulator (e.g. tegaserod), SGLT-2 inhibitor (e.g. empagliflozin), skeletal muscle relaxant (e.g. onabotulinumtoxinA), smoking quitting agent (e.g. nicotine analogue somatostat) (e.g. octreotide), statin (e.g. lovastatin), streptogramin (e.g. dalfopristin/quinupristin), streptomyces derivative (e.g. capreomycin), anticonvulsant succinimide (e.g. ethosuximide), sulfonamide (e.g. sulfamethoxazole), sulphonylurea stimulant (e.g. clomiphene), tetracyclic antidepressant (e.g. mirtazapine), tetracyclines (e.g. minocycline), thiazide diuretic (e.g. hydrochlorothiazide), thiazolidinedione (e.g. pioglitazone), thioxanthene (e.g. thiotixene), third generation cephalosporine (e.g. ceftriaxone), thrombin inhibitor (e.g. dabigatazone), strepolytic (e.g. levothyroxine), TNF alpha inhibitor (e.g. adalimumab), tocolytic agent (e.g. terbutaline), topical acne agent (e.g. tretinoin), topical anesthetic (e.g. lidocaine), topical anti-infection agent (e.g. malathion), topical anti-rosacea agent (e.g. ivermectin), topical antibiotic (e.g. silver sulfadiazine), topical antifungal (e.g. econazole), topical antihistamine (e.g. diphenhydramine), topical antineoplastic (e.g. imiquimod), topical anti psoriasis (e.g. tazarotene), topical antivirals (e.g. penciclovir), topical astringent (e.g. hazelnut), topical debridement agent (e.g. collagenase), topical depigmenting agent (e.g. hydroquinone), topical emollient (e.g. emollients), topical keratolytics (e.g. salicylic acid), topical non-steroidal anti-inflammatory (e.g. diclofenac), topical photochemistry (e.g. aminolevulinic acid), topical rubefactive (e.g. menthol), topical steroid (e.g. betamethasone), topical steroid with anti-infectives (e.g. aciclovir/hydrocortisone), transthyretin stabilizer (e.g. tafamidis), triazine anticonvulsant (e.g. lamotrigine), tricyclic antidepressant (e.g. amitriptyline), urea cycle disturbing agent (e.g. sodium phenylbutyrate), urinary anti-infective (e.g. nitrofurantoin), urinary antispasmodic (e.g. amitriptyline) modifier (e.g. potassium citrate), uterotonic agent (e.g. dinoprostone), vaginal anti-infective (e.g. clindamycin), vasodilator (e.g. alprostadil), vasopressin antagonist (e.g. conivaptan), vasopressor (e.g. epinephrine), VEGF/VEGFR inhibitor (e.g. pazopan), viral vaccine, combination of vitamins and minerals, vitamin (e.g. cyanocobalamin), VMAT2 inhibitor (e.g. valbenazine).

The nutritional active ingredient can be of the following types: vitamin (e.g. vitamins A, B, C, D, E, K, folic acid, biotin), mineral (e.g. potassium, chlorine, sodium, calcium, phosphorus, magnesium, iron, zinc, manganese, copper, iodine, chromium, molybdenum, selenium, cobalt, fluoride), amino acid, peptide and protein and their metabolites and derivatives (e.g. essential and branched amino acids, carnosine, enzymes and enzyme complexes, lactoferrin, N-acetylcysteine, proteins from animal or vegetable foods), fatty acid (e.g. omega-3, omega-6, omega-9 fatty acids), natural product manufactured using intact sources or substances extracted or derived from plants, animals, algae, fungi, lichens or bacteria (e.g. phytosterol, echinacea, green tea extract, garlic, aloe vera, fish oil, spirulina, chlorella, digestive enzymes derived from mushrooms), sugar and polysaccharides (e.g. mannose, ribose, trehalose, dextrose, glucuronolactone, dextrins), probiotic (e.g. live microorganisms such as Lactobacillus spp, Bifidobacterium spp, Sacc boulardii), prebiotic (e.g. fructans such as fructooligosaccharides and inulins, galactans such as galactooligosaccharides and xylooligosaccharides), antioxidant (e.g. lipoic acid, coenzyme Q10, flavonoids, glutathione, resveratrol, catechins), other substances with a nutritional or physiological effect (e.g. betaine, caffeine, theobromine, theophylline, CDP-choline, choline, creatine, phospholipids, GABA, glucosamine, inositol, melatonin, methylsulfonylmethane, nucleotides, squalene).

The inclusion of the active ingredient in the electrospun fiber can be obtained by co-electrospinning the active ingredient with the first compound. In this case, a mixture of the first compound to be electrospun and of the electrospinning promoter with the active ingredient can be prepared, and the mixture obtained is electrospun.

Alternatively, it can be provided to electrospin the first compound on its own, and subsequently to integrate the active ingredient in the fiber obtained. Depending on applications, it can be provided that the active ingredient is absorbed into the electrospun fiber, or that it is trapped in the three-dimensional structure obtained with the electrospun fiber.

For example, the first compound to be electrospun can comprise a mixture of linear hyaluronic acid with cross-linked hyaluronic acid. The cross-linked hyaluronic acid has the effect of increasing the rigidity of the nanometric fibers obtained, but also of increasing the complexity of the three-dimensional structure of a film obtained by continuously depositing the fibers obtained on several layers. In particular, the presence of cross-linked hyaluronic acid causes the formation of cavities in the film, cavities that allow to house the molecules of the active ingredient.

According to another example, the active ingredient is a non-steroidal anti-inflammatory, to be applied for example on a skin burn. It is also possible to provide to add one or more analgesics as additional active ingredients, in order to relieve the pain caused by the burn. For this type of application, it is particularly advantageous that the first compound is of the type that is regenerating for the skin, such as hyaluronic acid.

The use of the composition according to the invention in the treatment of skin burns is advantageous since it determines a fast absorption of the active ingredient and also of the first compound in the wound. In addition, the product able to be obtained by electrospinning the composition can be applied directly onto the burned zone. This improves the effectiveness of the treatment.

Advantageously, the active ingredient is present at a concentration comprised between 0.1% and 30% by weight, more preferably between 0.2% and 20% by weight, even more preferably between 0.5% and 10% by weight.

It should be noted that hyaluronic acid, as a compound to be electrospun, is a good candidate to be combined with different active ingredients, of each of the three types indicated above.

For example, among the cosmetic active ingredients we can mention the following: anti-seborrheic (sebacic acid, azelaic acid), antioxidants (ascorbic acid, tocopherol, retinol, retinal), anti-stain (glabridin, ammonium persulfate), emollients (Hamamelis virginiana extract, bisabolol) and humectants (e.g. glycerin, propanediol)

Among the preferred nutritional active ingredients are natural products manufactured using intact sources or substances extracted or derived from plants, animals, algae, fungi, lichens or bacteria (phytosterol, echinacea, green tea extract, garlic, aloe vera, fish oil, spirulina, chlorella, digestive enzymes derived from fungi), vitamins (e.g. vitamins A, B, C, D, E, K, folic acid, biotin) and antioxidants (e.g. lipoic acid, coenzyme Q10, flavonoids, glutathione, resveratrol, catechins).

The most beneficial pharmaceutical active ingredients are androgens and anabolic steroids (e.g. testosterone), anti-CTLA-4 monoclonal antibodies (e.g. ipilimumab), anti-PD-1 monoclonal antibodies (e.g. nivolumab), antianginal agents (e.g. nitroglycerin), anti-asthma combinations (e.g. diphyllin/guaifenesin), antibiotics (e.g. metronidazole), antibiotics/antineoplastics (e.g. doxorubicin), antineoplastics (e.g. isotretinoin) and antineoplastic combinations (e.g. letrozole/ribociclib).

It should be noted that these active ingredients can be advantageously combined with other compounds to be electrospun such as, for example, xanthan gum, guar gum, chondroitin sulfate, collagen or starch.

Another example of composition provides heparin as a compound to be electrospun and a pharmaceutical active ingredient, for example an allergen extract or a platelet stimulating agent such as eltrombopag.

According to some embodiments, the composition also comprises a stabilizer, which is preferably a cross-linkable polymer. One example of a stabilizer is sodium alginate, which is added to pullulan as a promoter. Preferably, the proportion of pullulan:alginate is comprised between 3:1.5 and 3:0.5, more preferably it is equal to 3:1.

### EXAMPLES

Electrospinning tests were performed on examples of the composition according to the present description. In the composition examples, the first compound to be electrospun is selected from the compounds listed in the table below:

| | |
|---|---|
| HA1 | linear hyaluronic acid with average molecular mass equal to 1.2 MDa |
| HA2 | hyaluronic acid oligomer with average molecular mass lower than 10000 Da |
| HA3 | hyaluronic acid with average molecular mass equal to 50000 Da |
| HA4 | cross-linked hyaluronic acid with average molecular mass comprised between 20 and 3000 kDa |

These compounds are supplied by Esperis S.p.A., Milan, Evonik Degussa Italia, Cremona, and IRALAB S.p.A., Usmate Velate (MI). Molecular masses were determined by means of GPC (Gel Permeation Chromatography).

The electrospinning was performed in a NANON.01A apparatus of the Japanese company Mecc CO. Ltd. The experimental conditions are indicated in each of the examples below.

The fibers produced were characterized by means of scanning electron microscopy. In particular, they were coated with gold using an EMITECHK950x Turbo Evaporator sputter coater, EBSciences, East Granby, CT, and observed with a Cambridge Stereoscan 440 SEM, Cambridge, UK scanning electron microscope.

### (Comparative example: PEO used as processing aid)

The spinning promoter comprises a mixture of an aqueous solution of alginate at 5% by weight, with an aqueous solution of PEO at 5% by weight in a proportion of 1:1. The promoter was then mixed with an aqueous solution of linear hyaluronic acid with an average molecular weight of 1.2 MDa at 0.5% by weight. The promoter:(HA solution) proportion is equal to 5.6:1. The composition was electrospun with relative humidity (RH) comprised between 24% and 29%, at a temperature of 22°C, with an electric field of 20kV, at a volumetric flow rate of the composition at the head equal to 0.7 mL/h, the distance between the spinning head and the support on which the fiber is deposited is equal to 15cm and the needle used being a 22G type needle. The fiber obtained is regular and has few defects. The same promoter was mixed with an aqueous solution of hyaluronic acid oligomer at 13% by weight, in a promoter:(HA solution) proportion equal to 1:3. The electrospinning of this second example of composition, under the same operating conditions as the first example as above, has a very regular and defect-free fiber, with an average diameter comprised between 250 and 350 nm. The fiber obtained completely covered the support used.

### Examples of electrospinning of compositions comprising hyaluronic acid as compound to be electrospun and pullulan as an spinning promoter

The pullulan used is of the food grade type, produced by Hayashibara Co., Ltd. Aqueous solutions of pullulan at 10%, 15% or 20% by weight were prepared, and these aqueous solutions of pullulan (spinning promoter) were mixed with aqueous solutions of hyaluronic acid, for the electrospinning.

The table below lists the examples of compositions that were electrospun, as well as the corresponding operating electrospinning conditions.

| | Composition | Electrospinning conditions |
|---|---|---|
| 1 | Pullulan 20%:HA3 29% 1:2 | RH 20-30%, 23kV, 0.1µl/min, 15 cm, needle 22G |
| 2 | Pullulan 20%:HA2 29% 1:2 | RH 46%, T=23°C, 23kV, 1ml/h, 15 cm, needle 22G |
| 3 | Pullulan 10%:HA2 23% 1:3 | RH 40%, T=24°C, 23-25kV, 1ml/h, needle 22G, max distance |
| 4 | Pullulan 10%:HA2 15% 1:2 | RH 49%, T=21°C, 23kV, 0.6ml/h, 18 cm, needle 22G, acid pH (between 1.5 and 3) |
| 5 | (pullulan 15%/alginate 5% 3:1):HA2 23% 1:3 | RH 49%, T=21°C, 23kV, 0.6ml/h, 15 cm, needle 22G |
| 6 | Pullulan 10%:HA2 23% 1:3 | RH 49%, T=21°C, 23kV, 0.15ml/h, 15 cm, needle 22G, acid pH (between 1.5 and 3) |
| 7 | Pullulan 10%:HA2 15% 1:2 | RH 40-50%, T=21°C, 23kV, 0.6ml/h, 15 cm, needle 22G, pH = 5.5 |
| 8 | Pullulan 10%:HA2 23% 1:3 | RH 30%, T=22°C, 23kV, 0.5ml/h, 15 cm, needle 22G, pH = 5.5 |

Example 1 resulted in regular fibers, without defects and with an average diameter from 400 to 700 nm. However, little deposit was observed during the test.

In example 2, the fibers obtained are thick, with an average diameter of 10 µm, due to the high viscosity of the electrospun solution.

In example 3, the fibers obtained have an average diameter comprised between 50 nm and 2 µm. It should be observed that with this example the fibers were deposited both on aluminum and also on a film of PBSA.

Examples 4 and 7 (pullulan:HA ratio equal to 1:2), on the one hand, and 6 and 8 (pullulan:HA ratio equal to 1:3), on the other hand, allowed to verify the effect of the proportions between promoter and hyaluronic acid. In example 4, the solution obtained has optimal properties for a good electrospinning, the fibers obtained have an average diameter ranging from 800 nm to 1 µm. For the composition of example 4, which has an acid pH, the pH was increased up to 5.5 (by adding NaOH 1M) thus obtaining the solution of example 7. With the latter, the electrospinning gave regular and uniform fibers with an average diameter smaller than in example 4, between 500 and 700 nm.

By increasing the proportion of hyaluronic acid, in example 6 (with acid pH) fibers with uniform diameter were obtained, with an average value equal to 1-3 µm, while in example 8 (with pH 5.5) the fibers obtained have a non-uniform diameter ranging from 700 nm to 3 µm.

In example 5, an alginate was added to the pullulan as a stabilizer. With a promoter:HA ratio of 1:3, and under the conditions mentioned in the table, thick fibers were obtained, with an average diameter in the order of several microns.

## Claims

1. Composition to be electrospun, comprising a first compound to be electrospun, an electrospinning promoter and at least one active ingredient, **characterized in that** said first compound to be electrospun is hyaluronic acid; said electrospinning promoter is selected from alginate, pullulan and a mixture thereof; and said active ingredient is selected from cosmetic active ingredients, pharmaceutical active ingredients and/or nutritional active ingredients.

2. Composition as in claim 1, **characterized in that** the proportion between first compound to be electrospun and electrospinning promoter is comprised between 10:1 and 1:10 by weight.

3. Composition as in any claim hereinbefore, **characterized in that** the first compound to be electrospun is diluted in an aqueous or aqueous-based solution at a concentration comprised between 0.1% and 10% by weight on the total weight of the composition.

4. Composition as in any claim hereinbefore, **characterized in that** the at least one active ingredient is present at a concentration comprised between 0.1% and 30% by weight on the total weight of the composition.

5. Method to prepare a composition to be electrospun, comprising a step of mixing a first compound to be electrospun with an electrospinning promoter, and a step of adding at least one active ingredient, wherein the first compound to be electrospun is hyaluronic acid, the electrospinning promoter is selected from alginate, pullulan and a mixture thereof, and the active ingredient is selected from cosmetic active ingredients, pharmaceutical active ingredients and nutritional active ingredients.

6. Method as in claim 5, **characterized in that** the step of adding the at least one active ingredient is performed at the same time as the step of mixing the first compound to be electrospun and the electrospinning promoter, **and in that** said at least one active ingredient is mixed with said first compound to be electrospun and said electrospinning promoter.

7. Method as in claim 5, **characterized in that** the step of adding the at least one active ingredient is performed after the step of mixing the first compound to be electrospun and the electrospinning promoter.

8. Composition to be electrospun as in any claim from 1 to 4 for use in the treatment of skin burns, wherein the active ingredient comprises a non-steroidal anti-inflammatory and/or one or more analgesics.

## Patentansprüche

1. Zusammensetzung, die elektrogesponnen werden soll, umfassend eine erste Verbindung, die elektrogesponnen werden soll, einen Elektrospinnpromotor und zumindest einen Wirkstoff, **dadurch gekennzeichnet, dass** die erste Verbindung, die elektrogesponnen werden soll, Hyaluronsäure ist; der Elektrospinnpromotor aus Alginat, Pullulan und einer Mischung davon ausgewählt ist; und der Wirkstoff aus kosmetischen Wirkstoffen, pharmazeutischen Wirkstoffen und/oder Ernährungswirkstoffen ausgewählt ist.

2. Zusammensetzung nach Anspruch 1, **dadurch gekennzeichnet, dass** das Verhältnis zwischen der ersten Verbindung, die elektrogesponnen werden soll, und dem Elektrospinnpromotor zwischen 10:1 und 1:10 gewichtsmäßig liegt.

3. Zusammensetzung nach einem der vorhergehenden Ansprüche, **dadurch gekennzeichnet, dass** die erste Verbindung, die elektrogesponnen werden soll, in einer wässrigen Lösung oder einer Lösung auf Wasserbasis in einer Konzentration zwischen 0.1% und 10% gewichtsmäßig bezogen auf das Gesamtgewicht der Zusammensetzung verdünnt ist.

4. Zusammensetzung nach einem der vorhergehenden Ansprüche, **dadurch gekennzeichnet, dass** der zumindest eine Wirkstoff in einer Konzentration zwischen 0.1% und 30% gewichtsmäßig bezogen auf das Gesamtgewicht der Zusammensetzung vorhanden ist.

5. Verfahren zur Zubereitung einer Zusammensetzung, die elektrogesponnen werden soll, umfassend einen Schritt des Vermischens einer ersten Verbindung, die elektrogesponnen werden soll, mit einem Elektrospinnpromotor und einen Schritt des Hinzufügens zumindest eines Wirkstoffs, worin die erste Verbindung, die elektrogesponnen werden soll, Hyaluronsäure ist, der Elektrospinnpromotor aus Alginat, Pullulan und einer Mischung davon ausgewählt ist, und der Wirkstoff aus kosmetischen Wirkstoffen, pharmazeutischen Wirkstoffen und Ernährungswirkstoffen ausgewählt ist.

6. Verfahren nach Anspruch 5, **dadurch gekennzeichnet, dass** der Schritt des Hinzufügens des zumindest einen Wirkstoffs zur gleichen Zeit durchgeführt wird wie der Schritt des Vermischens der ersten Verbindung, die elektrogesponnen werden soll, und des Elektrospinnpromotors, **und dass** der zumindest eine Wirkstoff mit der ersten Verbindung, die elektrogesponnen werden soll, und dem Elektrospinnpromotor vermischt wird.

7. Verfahren nach Anspruch 5, **dadurch gekennzeichnet, dass** der Schritt des Hinzufügens des zumindest einen Wirkstoffs nach dem Schritt des Vermischens der ersten Verbindung, die elektrogesponnen werden soll, und des Elektrospinnpromotors durchgeführt wird.

8. Zusammensetzung, die elektrogesponnen werden soll, nach einem der Ansprüche 1 bis 4 zur Verwendung bei der Behandlung von Hautverbrennungen, worin der Wirkstoff einen nichtsteroidalen Entzündungshemmer und/oder ein oder mehrere Schmerzmittel umfasst.

## Revendications

1. Composition à électrofiler, comprenant un premier composé à électrofiler, un promoteur d'électrofilage et au moins une substance active, **caractérisée en ce que** ledit premier composé à électrofiler est l'acide hyaluronique ; ledit promoteur d'électrofilage est sélectionné parmi l'alginate, le pullulane et un mélange de ceux-ci ; et ladite substance active est sélectionnée parmi des substances actives cosmétiques, des principes actifs pharmaceutiques et/ou des substances actives nutritionnelles.

2. Composition selon la revendication 1, **caractérisée en ce que** le rapport entre le premier composé à électrofiler et le promoteur d'électrofilage est compris entre 10:1 et 1:10 en poids.

3. Composition selon une quelconque revendication précédente, **caractérisée en ce que** le premier composé à électrofiler est dilué dans une solution aqueuse ou à base aqueuse à une concentration comprise entre 0.1% et 10% en poids par rapport au poids total de la composition.

4. Composition selon une quelconque revendication précédente, **caractérisée en ce que** la au moins une substance active est présente à une concentration comprise entre 0.1% et 30% en poids par rapport au poids total de la composition.

5. Procédé de préparation d'une composition à électrofiler, comprenant une étape de mélange d'un premier composé à électrofiler avec un promoteur d'électrofilage, et une étape d'ajout d'au moins une substance active, dans lequel le premier composé à électrofiler est l'acide hyaluronique, le promoteur d'électrofilage est sélectionné parmi l'alginate, le pullulane et un mélange de ceux-ci, et la substance active est sélectionnée parmi des substances actives cosmétiques, des principes actifs pharmaceutiques et des substances actives nutritionnelles.

6. Procédé selon la revendication 5, **caractérisé en ce que** l'étape d'ajout de l'au moins une substance active est effectuée en même temps que l'étape de mélange du premier composé à électrofiler et du promoteur d'électrofilage, **et en ce que** ladite au moins une substance active est mélangée avec ledit premier composé à électrofiler et ledit promoteur d'électrofilage.

7. Procédé selon la revendication 5, **caractérisé en ce que** l'étape d'ajout de l'au moins une substance active est effectuée après l'étape de mélange du premier composé à électrofiler et du promoteur d'électrofilage.

8. Composition à électrofiler selon l'une quelconque des revendications 1 à 4, pour utilisation dans le traitement des brûlures de la peau, dans laquelle la substance active comprend un anti-inflammatoire non stéroïdien et/ou un ou plusieurs analgésiques.
